(19) Europäisches Patentamt
European Patent Office
Office européen des brevets

(11) Veröffentlichungsnummer: **0 085 880**
**B1**

(12) **EUROPÄISCHE PATENTSCHRIFT**

(45) Veröffentlichungstag der Patentschrift: **11.09.85**

(51) Int. Cl.⁴ **C 07 C 102/04,** C 07 C 103/58, C 07 C 103/64

(21) Anmeldenummer: **83100610.1**

(22) Anmeldetag: **25.01.83**

(54) **Verfahren zur Herstellung von Ethen-(1,2)-dicarbonamidsäureestern.**

(30) Priorität: **06.02.82 DE 3204129**

(43) Veröffentlichungstag der Anmeldung: **17.08.83 Patentblatt 83/33**

(45) Bekanntmachung des Hinweises auf die Patenterteilung: **11.09.85 Patentblatt 85/37**

(84) Benannte Vertragsstaaten: **DE FR GB IT NL**

(56) Entgegenhaltungen:
**EP - A - 0 033 477**
**FR - A - 2 291 999**
**FR - A - 2 350 330**
**US - A - 3 228 972**

(73) Patentinhaber: **BAYER AG, Konzernverwaltung RP Patentabteilung, D-5090 Leverkusen 1 Bayerwerk (DE)**

(72) Erfinder: **Zecher, Wilfried, Dr., Treptower Strasse 6, D-5090 Leverkusen 1 (DE)**
Erfinder: **Merten, Rudolf, Dr., Berta-von-Suttner-Strasse 55, D-5090 Leverkusen 1 (DE)**

**EP 0 085 880 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es ist bekannt, daß man Malein- und Fumar-amidsäureester erhält, indem Malein- und Fumar-amidsäuren mit Alkoholen umgesetzt werden (Beilst. 12, S. 305 und 306). Malein- und Fumar-amidsäureester sind Ausgangsmaterialien für die Herstellung von Hydantoinen.

Aus der FR-PS 2 350 330 ist z. B. bekannt, daß die Umsetzung von aliphatischen Dicarbonsäuremonoestern mit Isocyanaten gesättigte Dicarbonamidsäureester liefert.

Weiterhin ist aus der EP-A-0 033 477 bekannt, daß die Umsetzung von Isocyanaten mit Ethen-(1,2)-dicarbonsäuremonoestern zu Hydantoinen führt.

Monomolekulare Hydantoine finden im Pharmabereich und auf dem Pflanzenschutzsektor Verwendung. Polyhydantoine können z. B. als temperaturbeständige Kunststoffe, insbesondere auf dem Elektroisoliersektor, verwendet werden (FR-PS 1 484 694).

Es wurde nun gefunden, daß man Ethen-(1,2)-dicarbonamidsäureester in guten Ausbeuten erhält, wenn Ethen-(1,2)-dicarbonsäuremonoester mit bis zu 1 Val eines organischen Isocyanates bei Temperaturen von 0—230°, vorzugsweise von 50—160°, umgesetzt werden.

Im erfindungsgemäßen Verfahren können Ethen-(1,2)-dicarbonsäuremonoester der Formel (I)

$$\left( R^3{-}OOC{-}\overset{\displaystyle R^1 \atop |}{C}{=}\overset{\displaystyle R^2 \atop |}{C}{-}COOH \right)_n \qquad (I)$$

in welcher

| | |
|---|---|
| $R^1$, $R^2$ und $R^3$ | gleich oder verschieden einen aliphatischen, aliphatisch-aromatischen oder aromatischen Rest, |
| $R^1$ und $R^2$ | außerdem noch Wasserstoff oder Halogen und |
| n | die Zahl 1, 2, 3 oder 4<br>bedeuten, |

eingesetzt werden.

Die Ethen-(1,2)-dicarbonsäuremonoester der Formel (I) können in der cis- oder trans-Form vorliegen. Vorzugsweise werden die trans-Ethen-(1,2)-dicarbonsäureester verwendet.

Die erfindungsgemäß als Ausgangsmaterialien verwendeten ungesättigten Dicarbonsäure-monoester sind z. B. aus Maleinsäureanhydrid und Alkoholen durch partielle Veresterung von Ethen-(1,2)-dicarbonsäuren oder durch partielle Verseifung der entsprechenden Diester zugänglich.

In Formel (I) bedeuten vorzugsweise

| | |
|---|---|
| $R^1$, $R^2$ und $R^3$ | gleich oder verschieden einen aliphatischen Rest mit 1 bis 20 C-Atomen, aliphatisch-aromatischen Rest mit 7 bis 15 C-Atomen oder aromatischen Rest mit 6 bis 16 C-Atomen, |
| $R^1$ und $R^2$ | außerdem noch unabhängig voneinander Wasserstoff, Fluor, Chlor oder Brom und |
| n | die Zahl 1 oder 2. |

Die Reste $R^1$ und $R^2$ leiten sich beispielsweise von Wasserstoff, Fluor, Chlor, Brom, Methan, Ethan, Hexan, Cyclohexan, Propen, Toluol und Benzol ab und können weiterhin zusammen einen Ring mit bis zu acht Kohlenstoffatomen bilden.

$R^3$ leitet sich beispielsweise von Methan, Ethan, n-, iso-, tert.-Butan, Hexan, Eicosan, Propen, Butin, Cyclohexan, Benzol, Naphthalin, Diphenylmethan, Diphenylether, Diphenylsulfon, $\omega$- oder kernsubstituiertem Toluol, Xylol, Polyethern, Polyestern, Polyharnstoffen und Polyurethanen ab und kann einfach oder mehrfach, z. B. mit Halogen wie Chlor und Brom oder Alkyl-, Carbonsäure-, Carbonestergruppen mit 1 bis 6 C-Atomen, Hydroxy- und Amino-Gruppen substituiert sein.

Besonders bevorzugt sind Monoester der Fumarsäure, in denen $R^3$ einen Alkan-Rest mit 1 bis 20 C-Atomen, einen Alken-Rest mit 2 bis 20 C-Atomen oder einen Alkin-Rest mit 3 bis 8 C-Atomen bedeutet.

Als Monoisocyanate im Sinne der Erfindung können aliphatische und aromatische, gegebenenfalls durch Heteroatome substituierte Verbindungen mit einer NCO-Gruppe im Molekül eingesetzt werden, z. B. Alkylisocyanate wie Ethyl-, Methyl-, Butyl-, Dodecyl- und Stearylisocyanat, aromatische, gegebenenfalls substituierte Monoisocyanate wie Phenyl-, Tolyl-, Isopropyl-, Nonylisocyanat, Nitro-, Alkoxy-, Aroxy-, Chlor-, Dichlor-, Trichlor-, Tetra-, Pentachlor-, Benzyl-, Brom-phenyl-isocyanat oder Isocyanatobenzoesäureester, Phthalsäureester, Isophthalsäureester, Isocyanatobenzonitril, cycloaliphatische Isocyanate wie Cyclohexylisocyanat und ungesättigte Isocyanate wie Allyl-, Oleyl-, Cyclohexenyl-isocyanat.

Als erfindungsgemäß einsetzbare Ausgangskomponenten können weiterhin aliphatische, cycloaliphatische, araliphatische, aromatische und heterocyclische Polyisocyanate, vorzugsweise Diisocyanate in Betracht kommen, (vgl. Annalen, 562, Seite 75 bis 136), beispielsweise Ethylendiisocyanat,

1,4-Tetramethylendiisocyanat, 1,6-Hexamethylendiisocyanat, 1,12-Dodecandiisocyanat, Cyclobutan-1,3-diisocyanat, Cyclohexan-1,3- und -1,4-diisocyanat sowie beliebige Gemische dieser Isomeren, 1-Isocyanato-3,3,5-trimethyl-5-isocyanato-methyl-cyclohexan (z. B. DE-AS 1 202 785), 2,4- und 2,6-Hexahydrotoluylendiisocyanat sowie beliebige Gemische dieser Isomeren, Hexahydro-1,3- und/oder -1,4-phenylen-diisocyanat, Perhydro-2,4'- und/oder -4,4'-diphenylmethan-diisocyanat, 1,3- und 1,4-Phenylendiisocyanat, 2,4- und 2,6-Toluylendiisocyanat sowie beliebige Gemische dieser Isomeren, Diphenylmethan-2,4'- und/oder -4,4'-diisocyanat, Naphthylen-1,5-diisocyanat, Triphenylmethan-4,4'-,4''-Triisocyanat, Polyphenyl-polymethylen-polyisocyanate, wie sie z. B. durch Anilin-Formaldehyd-Kondensation und anschließende Phosgenierung erhalten werden (z. B. GB-PS 874 430 und 848 671), perchlorierte Arylpolyisocyanate, (z. B. DE-AS 1 157 601), Carbodiimidgruppen aufweisende Polyisocyanate (z. B. DE-PS 1 092 007), Diisocyanate (z. B. US-PS 3 492 330), Allophanatgruppen aufweisende Polyisocyanate (z. B. BE-PS 761 626 und NL-A 7 102 524), Isocyanuratgruppen aufweisende Polyisocyanate, z. B. DE-PS 1 022 789, 1 222 067 und 1 027 394, DE-OS 1 929 034 und 2 004 048), Urethangruppen aufweisende Polyisocyanate (BE-PS 752 261, US-PS 3 394 164), acylierte Harnstoffgruppen aufweisende Polyisocyanate (z. B. 1 230 778), Biuretgruppen aufweisende Polyisocyanate (z. B. DE-PS 1 101 394, GB-PS 889 050, FR-PS 7 017 514), durch Telomerisationsreaktionen hergestellte Polyisocyanate (z. B. BE-PS 723 640), Estergruppen aufweisende Polyisocyanate, (z. B. GB-PS 956 474 und 1 072 956, US-PS 3 567 763, DE-PS 1 231 688), Umsetzungsprodukte der obengenannten Isocyanate mit Acetylen (z. B. DE-PS 1 072 358).

Es ist auch möglich, die bei der technischen Isocyanatherstellung anfallenden Isocyanatgruppen aufweisenden Destillationsrückstände, gegebenenfalls gelöst in einem oder mehreren der genannten Polyisocyanate, einzusetzen. Weiterhin ist es möglich, beliebige Mischungen der vorgenannten Polyisocyanate zu verwenden.

Vorzugsweise eignen sich Mono- bzw. Polyiso(thio)cyanate der allgemeinen Formeln (II)

$R^4(-NCO)_z$ bzw. $R^4(-NCS)_z$ (II)

in welchen

$R^4$ für einen gegebenenfalls mit Halogen, Alkyl- und/oder Arylgruppen substituierten aliphatischen Rest mit 1—20 C-Atomen, einen aromatischen Rest mit 5—12 C-Atomen, einen cycloaliphatischen Rest mit 5—12 C-Atomen, einen aliphatischen-aromatischen Rest mit 6—20 C-Atomen und einen Heteroatome wie N, O oder S enthaltenden aromatischen oder cycloaliphatischen Rest mit 5—12 C-Atomen, steht. Besonders bevorzugt sind aliphatische Reste mit 2—12 C-Atomen, oder ein Arylrest wie Phenyl, Tolyl, Naphthyl, Dipentylmethan und Diphenyletherreste.

z ist eine ganze Zahl von 1—4, vorzugsweise 1—3, besonders bevorzugt 2.

Bevorzugt verwendet werden die technisch leicht zugänglichen Gemische aus Toluylen-diisocyanaten, m-Phenylendiisocyanat, Phenylisocyanat und seine Substitutionsprodukte, Methylisocyanat, sowie phosgenierte Kondensate aus Anilin und Formaldehyd mit Polyphenylenmethylenstruktur und die symmetrischen Verbindungen 4,4'-Diisocyanatodiphenylmethan, 4,4'-Diisocyanatodiphenylether, p-Phenylendiisocyanat, 4,4'-Diisocyanato-diphenyl-dimethylmethan, analoge Diisocyanate, die sich von den hydrierten Aromaten ableiteten, sowie aliphatische Diisocyanate mit 2—12 C-Atomen wie Hexamethylendiisocyanat und von Isophoron abgeleitete Diisocyanate.

Die Isocyanate können in freier Form eingesetzt werden. Weiterhin ist es auch möglich sie zum Teil oder vollständig in Form ihrer beim Umsatz mit reaktivem Wasserstoff enthaltenden Verbindungen zugänglichen und unter den Reaktionsbedingungen als Abspalter reagierenden Derivaten, einzusetzen.

Vorzugsweise werden als Abspalter die aus Lactamen, z. B. Caprolactam und Pyrrolidon zugänglichen Acyl-Harnstoffe und die aus aromatischen und aliphatischen Mono- und Polyhydroxy-Verbindungen erhaltenen Carbamidsäureester umgesetzt, die z. B. den allgemeinen Formeln (III)

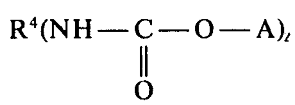 bzw. 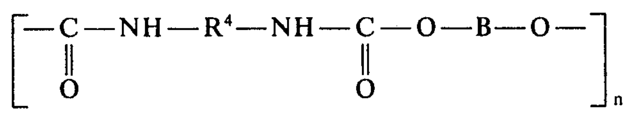 (III)

in denen

$R^4$ und z die bei Formel (II) angegebene Bedeutung haben und
A der organische Rest einer Monohydroxyverbindung bzw.
B der organische Rest einer bis- oder trisfunktionellen Hydroxyverbindung und
n für eine ganze Zahl von 1 bis 1000 steht,

entsprechen.

In den Formeln (III) stehen vorzugsweise

$R^4$ für die bei Formel (II) vorzugsweise angegebene Bedeutung,
A für einen organischen Rest einer Monohydroxyverbindung und
B für einen organischen Rest einer bis- oder trisfunktionellen Hydroxyverbindung, wobei sich A und B von aliphatischen Resten mit 1 bis 10 C-Atomen, cycloaliphatischen Resten mit 5 bis 10 C-Atomen, aliphatisch-aromatischen Resten mit 7 bis 12 C-Atomen und aromatischen Resten mit 6 bis 12 C-Atomen ableiten, die gegebenenfalls mit $C_1$ bis $C_4$ Alkylgruppen und/oder Arylgruppen mit 6 bis 10 C-Atomen substituiert sein können und
n für eine ganze Zahl von 1 bis 100.

Als Beispiele seien Carbamidsäureester von Phenol, isomerer Kresole, deren technische Gemische und ähnliche aromatische Hydroxylverbindungen, aliphatische Monoalkohole wie Methanol, Ethanol, Propanol, Isopropanol, Butanol, Isobutanol, Diethylenglykolmonomethylether, Cyclohexanol, Benzylalkohol und aliphatische Di- oder Polyole wie Ethylenglykol und Trimethylolpropan aufgeführt.

Die Urethane können als solche eingesetzt oder erst in situ durch Umsetzung mit Alkoholen erzeugt werden.

Anstelle der genannten (Poly)Isocyanate können auch die analogen (Poly)Isothiocyanate eingesetzt werden.

Durch das nachfolgende Reaktionsschema soll die erfindungsgemäße Umsetzung erläutert werden:

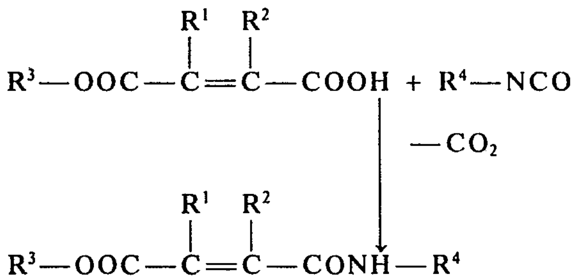


In der Reaktionsgleichung haben $R^1$, $R^2$, $R^3$ und $R^4$ die oben angegebene Bedeutung.

Die erfindungsgemäßen Amidester können neben den üblichen analytischen Verfahren durch ihre IR-Spektren an Hand der für Amide und Ester charakteristischen Banden identifiziert werden.

Die erfindungsgemäße Umsetzung kann in Lösungsmitteln, die unter den Reaktionsbedingungen inert sind oder nur lockere Additionsverbindungen bilden, ausgeführt werden. Es kann auch in einem Überschuß an Ethen-(1,2)-dicarbonamidsäureester umgesetzt werden. Geeignete Lösungsmittel sind beispielsweise:

(Halogen)-Kohlenwasserstoffe wie Tetrachlorethan, o-Dichlorbenzol, Alkylaromaten wie Xylole, Phenole wie Phenol, Ester wie Benzoesäurealkylester, Lactone wie Butyrolacton, Caprolacton, Ketone wie Acetophenon, Cyclohexanon, Ester wie Glykolmonomethyletheracetat, Ether wie Diethylenglykoldimethylether, Amide wie Dimethylformamid, N-Methylpyrrolidon, Lactame wie Caprolactam, Nitrile wie Benzonitril, Phosphorsäureamide wie Hexamethylphosphorsäuretriamid, Sulfoxide wie Dimethylsulfoxid und Sulfone wie Tetramethylensulfon. Es können Gemische der Lösungsmittel eingesetzt werden.

Die Durchführung des erfindungsgemäßen Verfahrens erfolgt in der Weise, daß die Umsetzungskomponenten mit oder ohne Lösungsmittel einige Minuten bis zu mehreren Stunden bei Temperaturen von 0—230°, vorzugsweise von 50—160° gehalten werden. Der Verlauf der Umsetzung kann über die Gasentwicklung und die IR-Spektren verfolgt werden.

Im allgemeinen werden pro Val Monoester 1 Val Isocyanat zur Umsetzung gebracht. Es können auch weniger als 1 Val Isocyanat eingesetzt werden.

In Abhängigkeit von der Funktionalität der erfindungsgemäßen Dicarbonsäuremonoester und der Isocyanate werden $\alpha,\beta$-ungesättigte Mono-, Oligo- und Poly-carbonamidsäureester erhalten.

Die erfindungsgemäße Umsetzung kann durch Katalysatoren beeinflußt werden. Beispielsweise können Amine wie Triethylamin und 1,4-Diaza-bicyclo-(2,2,2)-octan, organische und anorganische Metallverbindungen, insbesondere des Eisens, Bleis, Zinks, Zinns, Kupfers, Kobalts und Titans, wie Eisen(III)-chlorid, Kobaltacetat, Bleioxid, Zinkacetat, Zinnoctoat, Dibutyl-zinn-dilaurat, Kupfer-acetylacetonat, Titantetrabutylat, Alkaliphenolate und Natriumcyanid und Phosphorverbindungen wie Trialkylphosphin und Phospholinoxide eingesetzt werden.

Als besonders geeignet haben sich Alkyl- und Aryl-phospholinoxide wie Methyl-phospholinoxid erwiesen.

Die nach dem erfindungsgemäßen Verfahren herstellbaren Ethan-(1,2)-dicarbonamidsäureester zeigen Wirkungen auf dem Pflanzenschutz-Sektor und sind außerdem Ausgangsmaterialien für die Herstellung von Kunststoffen, z. B. von Hydantoinen, wie dies in der Deutschen Offenlegungsschrift 3 200 704 beschrieben wird.

Beispiel 1

In 260 g eines technischen Xylol-Gemisches werden 72 g Fumarsäuremonoethylester, 60 g Phenylisocyanat und 0,7 g Dibutylzinn-dilaurat als Katalysator jeweils 2 Stdn. auf 80, 100 und 120°C und 6 Stdn. auf 140°C erhitzt. Die Reaktion erfolgt unter Abspaltung von Kohlendioxid. Beim Erkalten kristallisiert der reine N-Phenylfumaramidsäureethylester in farblosen Kristallen vom Schmp. 107—108°. Aus der Mutterlauge werden nach dem Verdampfen des Xylols und der Umkristallisation des Rückstandes aus Ethanol weitere 9 g des Amidesters erhalten. Das IR-Spektrum zeigt bei 1650, 1680 und 1705 $cm^{-1}$ für $\alpha,\beta$-ungesättigte Carbonamidester charakteristische Banden.

$C_2H_5—OOC—CH=CH—CO—NH—Ph$

$C_{12}H_{13}NO_3$ (219):

| | | | |
|---|---|---|---|
| ber. | C 65,8 | H 5,9 | N 6,4%; |
| gef. | C 66,1 | H 6,2 | N 6,5%. |

Werden anstelle des Dibutyl-zinn-dilaurats als Katalysator 0,7 g Methylphospholinoxid (Gemisch aus 1-Methyl-1-phospha-2- und 1-Methyl-1-phospha-3-cyclopenten-(1)-oxid) eingesetzt, so beträgt die Ausbeute an reinem N-Phenylfumaramidsäureethylester 96 g entsprechend 88% der Theorie.

Beispiel 2

142 g Fumarsäuremonododecylester, 63 g Cyclohexylisocyanat und 0,5 g Methylphospholinoxid werden in 200 g Xylol eingetragen und jeweils 2 Stdn. bei 100 und 120°C und 6 Stdn. bei 140°C gerührt. Beim Erkalten kristallisiert das Reaktionsprodukt aus und wird abfiltriert. Nach der Umkristallisation aus Acetonitril erhält man den N-Cyclohexyl-fumaramidsäuredodecylester in farblosen Kristallen vom Schmp. 91—92°C mit IR-Banden bei 1635, 1660 und 1710 $cm^{-1}$.

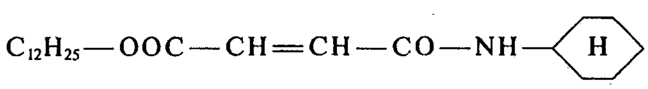

$C_{22}H_{39}NO_3$ (365):

| | | | |
|---|---|---|---|
| ber. | C 72,3 | H 10,7 | N 3,8%; |
| gef. | C 72,2 | H 11,0 | N 3,5%. |

Beispiel 3

In 270 g Xylol werden 158 g Fumarsäureisopropylester und 119 g Phenylisocyanat jeweils 4 Stdn. auf 110, 120, 130 und 140°C erhitzt. Der N-Phenyl-fumaramidsäureisopropylester kristallisiert beim Erkalten aus, wird abgetrennt und aus Cyclohexan umkristallisiert. Man erhält die reine Verbindung in farblosen Kristallen vom Schmp. 99—100°C.

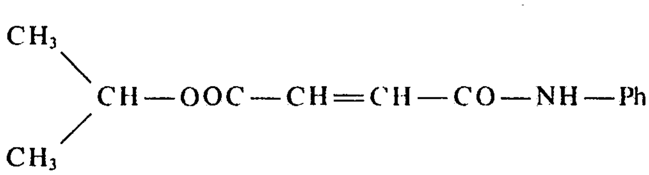

$C_{13}H_{15}NO_3$ (233):

| | | | |
|---|---|---|---|
| ber. | C 67,0 | H 6,4 | N 6,0%; |
| gef. | C 66,9 | H 6,4 | N 6,1%. |

Beispiel 4

In 135 g Dimethylacetamid werden 71,5 g Fumarsäuremonoethylester und 62,5 g 4,4'-Diisocyanato-diphenylmethan jeweils 4 Stdn. bei 80, 100 und 120° gerührt. Nach dem Erkalten wird mit Methanol gefällt und die Fällung aus Acetonitril umkristallisiert. Man erhält den Fumaramidsäureester in farblosen Kristallen mit dem Schmp. 226—227° und IR-Banden bei 1655, 1685 und 1725 $cm^{-1}$.

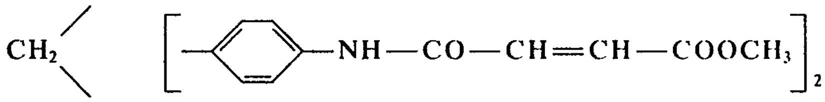

$C_{23}H_{22}N_2O_6$ (422):

ber. C 65,4 H 5,2 N 6,6%;
gef. C 65,3 H 4,9 N 6,9%.

Beispiel 5

In 220 g N-Methylpyrrolidon werden 130 g Fumarsäuremonomethylester, 87 g Toluylen-(2,4)-diisocyanat und 1 g Hydrochinon gelöst und in jeweils 4 Stdn. bei 80, 10 und 110°C und 2 Stdn. bei 120°C umgesetzt. Das Reaktionsgemisch wird in 3 kg Wasser eingetropft, die Fällung abgetrennt, getrocknet und mit Methylenchlorid verrührt. Der Rückstand ergibt bei der Umkristallisation aus Acetonitril/Dimethylformamid den Fumaramidsäureester in schwach gelben Kristallen vom Schmp. 213–214°.

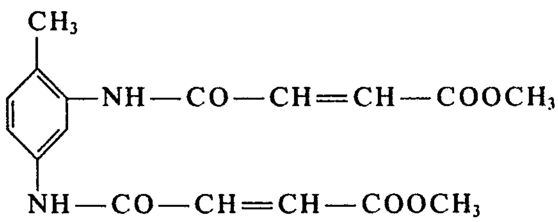

ber. C 59,0 H 5,2 N 8,1%;
gef. C 58,7 H 5,2 N 8,0%.

Beispiel 6

In 280 g N-Methylpyrrolidon werden 158 g Fumarsäuremonoisopropylester und 125 g 4,4'-Diisocyanato-diphenylmethan in jeweils 3 Stdn. bei 80, 100 und 120°C umgesetzt. Das Reaktionsprodukt wird mit Wasser gefällt, abgetrennt, getrocknet, mit Essigsäureethylester ausgewaschen und dann aus Acetonitril umkristallisiert. Man erhält den Fumaramidsäureester in beigen Kristallen mit dem Schmp. 210–212°C und IR-Banden bei 1645, 1680 und 1725 $cm^{-1}$.

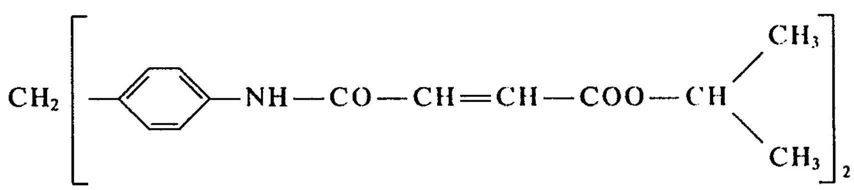

$C_{27}H_{30}N_2O_6$ (478):

ber. C 67,8 H 6,3 N 5,9%;
gef. C 67,8 H 6,4 N 6,2%.

Beispiel 7

In 210 g N-Methylpyrrolidon werden 130 g Fumarsäuremonomethylester mit 84 g Hexamethylendiisocyanat in jeweils 3 Stdn. bei 130, 150 und 170° umgesetzt. Der Fumaramidsäureester fällt beim Erkalten aus, wird abgetrennt und aus Acetonitril umkristallisiert. Man erhält farblose Kristalle vom Schmp. 192–193°C mit Banden im IR-Spektrum bei 1640, 1670 und 1725 $cm^{-1}$.

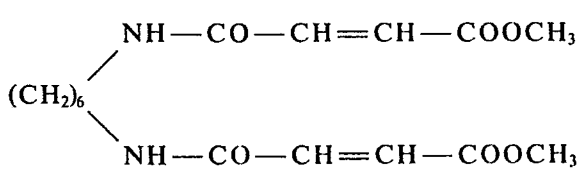

$C_{16}H_{24}N_2O_6$ (340):

| | | | |
|---|---|---|---|
| ber. | C 56,5 | H 7,1 | N 8,2%; |
| gef. | C 56,1 | H 7,1 | N 8,5%. |

Beispiel 8

In 400 g Xylol werden 144 g Fumarsäuremonoethylester mit 261 g Hexadecylisocyanat und 0,5 g Methylphospholinoxid als Katalysator in 5 Stdn. bei 150°C umgesetzt. Beim Erkalten kristallisiert der Fumaramidsäureester aus und wird abfiltriert. Die Umkristallisation zuerst aus Methanol und dann aus Acetonitril ergibt farblose Kristalle vom Schmp. 74–75°.

$$C_2H_5OOC-CH=CH-CO-NH-C_{16}H_{33}$$

$C_{22}H_{41}NO_3$ (367):

| | | | |
|---|---|---|---|
| ber. | C 71,9 | H 11,2 | N 3,8%; |
| gef. | C 71,9 | H 11,2 | N 3,9%. |

Beispiel 9

77 g Fumarsäuremonopropargylester, 59,5 g Phenylisocyanat und 0,7 g Methylphospholinoxid als Katalysator werden in 280 g Xylol jeweils 3 Stdn. bei 80, 100 und 120° gerührt. Beim Erkalten fällt der N-Phenylfumaramidsäurepropargylester aus und wird abfiltriert. Die Umkristallisation aus Toluol und dann aus Acetonitril ergibt farblose Kristalle vom Schmp. 166–167° mit IR-Banden bei 1650, 1675 und 1715 $cm^{-1}$.

$$HC\equiv C-CH_2-OOC-CH=CH-CO-NH-Ph$$

$C_{13}H_{11}NO_3$ (229):

| | | | |
|---|---|---|---|
| ber. | C 68,1 | H 4,8 | N 6,1%; |
| gef. | C 68,1 | H 4,8 | N 6,1%. |

Beispiel 10

77 g Fumarsäuremonopropargylester und 29 g Methylisocyanat werden in 280 g Xylol gelöst. Die Temperatur der Lösung wird, bei 40°C beginnend, im Verlaufe von 4 Stdn. auf 70°C gesteigert und dann noch 4 Stdn. in diesem Bereich gehalten.

Der N-Methyl-fumaramidsäurepropargylester kristallisiert beim Erkalten aus und wird abgetrennt. Aus der Mutterlauge wird nach dem Eindampfen ein weiterer Anteil erhalten. Die Umkristallisation aus Toluol und danach aus Essigsäureethylester ergibt farblose Kristalle vom Schmp. 102–103° mit Banden im IR-Spektrum bei 1630, 1655 und 1720 $cm^{-1}$.

$$HC\equiv C-CH_2-OOC-CH=CH-CO-NH-CH_3$$

$C_8H_9NO_3$ (167):

| | | | |
|---|---|---|---|
| ber. | C 57,5 | H 5,4 | N 8,4%; |
| gef. | C 57,7 | H 5,7 | N 8,4%. |

**Patentansprüche**

1. Verfahren zur Herstellung von Ethen-(1,2)-dicarbonamidsäureestern, dadurch gekennzeichnet, daß Ethen-(1,2)-dicarbonsäuremonoester mit bis zu 1 Val eines organischen Isocyanates bei Temperaturen von 0 bis 230° umgesetzt werden.

2. Verfahren nach Anspruch 1, dadurch gekennzeichnet, daß bei Temperaturen von 50 bis 160°C umgesetzt wird.

3. Verfahren nach Anspruch 1, dadurch gekennzeichnet, daß Ethen-(1,2)-dicarbonsäuremonoester der Formel (I)

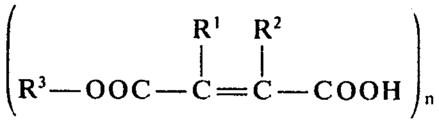 (I)

in welcher

| | |
|---|---|
| $R^1$, $R^2$ und $R^3$ | gleich oder verschieden einen aliphatischen, aliphatisch-aromatischen oder aromatischen Rest, |
| $R^1$ und $R^2$ | außerdem noch Wasserstoff oder Halogen und |
| n | die Zahl 1, 2, 3 oder 4 bedeuten, |

eingesetzt werden.

4. Verfahren nach Anspruch 1, dadurch gekennzeichnet, daß Ethen-(1,2)-dicarbonsäuremonoester der Formel (I) eingesetzt werden, in welcher

| | |
|---|---|
| $R^1$, $R^2$ und $R^3$ | gleich oder verschieden einen aliphatischen Rest mit 1 bis 20 C-Atomen, aliphatisch-aromatischen Rest mit 7 bis 15 C-Atomen oder aromatischen Rest mit 6 bis 16 C-Atomen, |
| $R^1$ und $R^2$ | außerdem noch unabhängig voneinander Wasserstoff, Fluor, Chlor oder Brom und |
| n | die Zahl 1 oder 2. |

bedeuten.

5. Verwendung von Ethen-(1,2)-dicarbonamidsäureester hergestellt nach Verfahren 1 zur Herstellung von Hydantoinen.

## Claims

1. Process for the preparation of ethene-1,2-dicarboxylic acid amide esters, characterised in that ethene-1,2-dicarboxylic acid monoesters are reacted with up to 1 equivalent of an organic isocyanate at temperatures of 0 to 230°.

2. Process according to Claim 1, characterised in that the reaction is carried out at temperatures of 50 to 160°C.

3. Process according to Claim 1, characterised in that ethene-1,2-dicarboxylic acid monoesters of the formula (I)

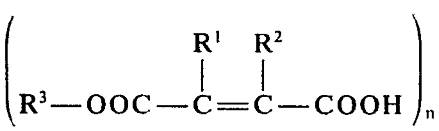 (I)

in which

| | |
|---|---|
| $R^1$, $R^2$ and $R^3$ | are identical or different and denote an aliphatic, aliphatic-aromatic or aromatic radical, |
| $R^1$ and $R^2$ | also denote hydrogen or halogen and |
| n | denotes the number 1, 2, 3 or 4 |

are used.

4. Process according to Claim 1, characterised in that ethene-1,2-dicarboxylic acid monoesters of the formula (I) are used, in which

| | |
|---|---|
| $R^1$, $R^2$ and $R^3$ | are identical or different and denote an aliphatic radical with 1 to 20 C atoms, an aliphatic-aromatic radical with 7 to 15 C atoms or an aromatic radical with 6 to 16 C atoms, |
| $R^1$ and $R^2$ | also, independently of one another, denote hydrogen, fluorine, chlorine or bromine, and |
| n | denotes the number 1 or 2. |

5. Use of ethene-1,2-dicarboxylic acid amide esters prepared according to process 1 for the production of hydantoins.

## Revendications

1. Procédé de production d'esters d'acide éthène-(1,2)-dicarboxamique, caractérisé en ce qu'on fait réagir un mono-ester d'acide éthène-(1,2)-dicarboxylique avec jusqu'à 1 valence d'un isocyanate

organique à des températures de 0 à 230°C.

2. Procédé suivant la revendication 1, caractérisé en ce qu'on conduit la réaction à des températures de 50 à 160°C.

3. Procédé suivant la revendication 1, caractérisé en ce qu'on utilise des mono-esters d'acide éthène-(1,2)-dicarboxylique de formule (I)

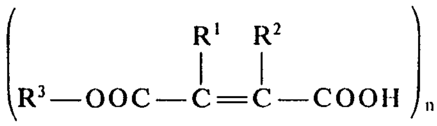

(I)

dans laquelle

| | |
|---|---|
| $R^1$, $R^2$ et $R^3$ | égaux ou différents, représentent un reste aliphatique, aliphato-aromatique ou aromatique, |
| $R^1$ et $R^2$ | représentent en outre l'hydrogène ou un halogène et |
| n | représente le nombre 1, 2, 3 ou 4 |

4. Procédé suivant la revendication 1, caractérisé en ce qu'on utilise des mono-esters d'acide éthène-(1,2)-dicarboxylique de formule (I) dans laquelle

| | |
|---|---|
| $R^1$, $R^2$ et $R^3$ | égaux ou différents, représentent un reste aliphatique ayant 1 à 20 atomes de carbone, un reste aliphato-aromatique ayant 7 à 15 atomes de carbone ou un reste aromatique ayant 6 à 16 atomes de carbone, |
| $R^1$ et $R^2$ | représentent en outre, indépendamment l'un de l'autre, l'hydrogène, le fluor, le chlore ou le brome et |
| n | représente le nombre 1 ou 2. |

5. Utilisation d'esters d'acide éthène-(1,2)-dicarboxamique obtenus par le procédé 1 pour la préparation d'hydantoïnes.